# EUROPEAN PATENT APPLICATION

(11) **EP 1 666 467 A1**
(43) Date of publication of application: **07.06.2006**
(21) Application number: 04026440.0
(22) Date of filing: 08.11.2004
(51) Int. Cl.: C07D 213/76, A61K 31/44, A61P 3/04, A61P 3/06, A61P 3/10

(54) **11Beta-HSD1 Inhibitors**

(71) Applicant: Evotec AG, 22525 Hamburg (DE); DeveloGen Aktiengesellschaft, 37079 Göttingen (DE)
(72) Inventor: Coulter, Thomas Stephen, Grove Bridge Wantage OX12 7PA (GB); Taylor, Steven, Didcot OX11 7AU (GB); Fryatt, Tara, Didcot OX11 7XU (GB); Aicher, Babette, 37073 Goettingen (DE); Schneider, Martin, 37081 Goettingen (DE)
(74) Representative: Isenbruck, Günter

(57) **Abstract**

The invention relates to sulfonamide compounds of formula (I) 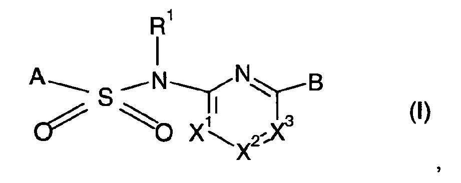 wherein A, B, R¹ and X¹ to X³ have the meaning as cited in the description and the claims. For example A is biphenyl, B is 2-morpholin-4-yl-2-oxo-ethyl, R¹ is H and X¹ to X³ are CH. Said compounds are useful as 11β-HSD1 inhibitors. The invention also relates to the preparation of such compounds as well as the production and use as medicament.

## Description

The present invention relates to a novel class of 11β-HSD 1 inhibitors, including pharmaceutically acceptable salts thereof, which are useful as therapeutic compounds, particularly in the treatment of type 2 diabetes mellitus, often referred to as non-insulin dependent diabetes mellitus (NIDDM), and of conditions that are often associated with this disease, such as metabolic syndrome, obesity and lipid disorders. The present invention also relates to processes for preparing said compounds.

Diabetes is a disease derived from multiple causative factors and characterized by elevated levels of plasma glucose (hyperglycemia) in the fasting state or after administration of glucose during an oral glucose tolerance test. There are two generally recognized forms of diabetes. In type 1 diabetes, or insulin-dependent diabetes mellitus (IDDM), patients produce little or no insulin, the hormone which regulates glucose utilization. In type 2 diabetes, or noninsulin-dependent diabetes mellitus (NIDDM), insulin is still produced in the body. Patients often have hyperinsulinemia (plasma insulin levels that are the same or even elevated in comparison with non-diabetic subjects); however, these patients have developed insulin resistance, which is a resistance to the effect of insulin in stimulating glucose and lipid metabolism in the main insulin-sensitive tissues, which are muscle, liver and adipose tissues. Patients who are insulin resistant but not diabetic have elevated insulin levels that compensate for the insulin resistance so that serum glucose levels are not elevated. In patients with NIDDM, the plasma insulin levels, even when they are elevated, are insufficient to overcome the pronounced insulin resistance.

Insulin resistance is not primarily due to a diminished number of insulin receptors but to a post-insulin receptor binding defect that is not yet completely understood. This resistance to insulin responsiveness results in insufficient insulin activation of glucose uptake, oxidation and storage in muscle and inadequate insulin repression of lipolysis in adipose tissue and of glucose production and secretion in the liver (gluconeogenesis and glycogenolysis).

Persistent or uncontrolled hyperglycemia that occurs with diabetes is associated with increased and premature morbidity and mortality. Often abnormal glucose homeostasis is associated both directly and indirectly with obesity, hypertension, and alterations of the lipid, lipoprotein and apolipoprotein metabolism and other metabolic and hemodynamic disease. Therefore patients with type 2 diabetes mellitus are at an especially increased risk of macrovascular and microvascular complications, including atherosclerosis, coronary heart disease, stroke, peripheral vascular disease, hypertension, nephropathy, neuropathy, and retinopathy.

Therefore, therapeutic control of glucose homeostasis, lipid metabolism, obesity, and hypertension are critically important in the clinical management and treatment of diabetes mellitus and metabolic syndrome.

Many patients who have insulin resistance but have not developed type 2 diabetes are at a risk of developing at least several symptoms selected from a group of symptoms that are often referred to as syndrome X, or the metabolic syndrome.

This syndrome is characterized by insulin resistance, abdominal/visceral obesity, hyperinsulinemia, high blood pressure, low HDL, and high VLDL (dyslipidemia). These patients, whether or not they develop overt diabetes mellitus, are at increased risk of the macrovascular and microvascular complications of type 2 diabetes listed above (e. g. atherosclerosis and coronary heart disease/cardiovascular disease).
The metabolic syndrome often starts with continuous weight increase leading to significant visceral obesity. Increased visceral adipose tissue mass, which is not only an energy storage organ but also an important endocrine organ secreting hormones and signalling molecules, is believed to be a major source for the development of all the diseases that are summarized as metabolic syndrome especially dyslipidemia, hypertension, insulin resistance and β-cell dysfunction. Progression of visceral obesity can lead toinsulin resistance causing impaired glucose tolerance and finally type 2 diabetes mellitus. Diabetes, dyslipidemia and hypertension are major risk factors for cardiovascular disease, a main cause for death in western world.
Increased 11 β-HSD 1 activity in adipocytes is believed to be one of the essential factors that are responsible for the increase of visceral adipose tissue mass because overexpression in adipose tissue in mice causes visceral obesity and finally a disease state representing human metabolic syndrome (Masuzaki, H. et al. 2001).Furthermore, increased 11β-HSD 1 activity in visceral adipose tissue is found in patients with visceral obesity and/or type 2 diabetes mellitus and in patients with metabolic syndrome.
On the other hand deletion of 11β-HSD 1 activity in mice protects against diet-induced obesity and related diseases like, glucose intolerance, insulin resistance, type 2 diabetes mellitus and metabolic syndrome (Kotelevtsev, Y. et al. 1997; Morton, NM. et al. 2001 and 2004) and ameliorates age-related learning impairments in addition (Yau, JLW. et al. 2001).

The available treatments for type 2 diabetes have not changed substantially in many years, and these treatments have recognized limitations.

Physical exercise and reductions in dietary intake of calories often dramatically improve the diabetic condition, but compliance with this treatment is very poor because of well-entrenched sedentary lifestyles and excess food consumption, especially of foods containing high amounts of sugar and saturated fat. Increasing the plasma level of insulin by administration of sulfonylureas (e. g. tolbutamide and glipizide) or meglitinide, which stimulate the pancreatic β-cells to secrete more insulin, and/or by injection of insulin when sulfonylureas or meglitinide become ineffective, can result in insulin concentrations high enough to stimulate the very insulin-resistant tissues.

However, dangerously low levels of plasma glucose can result from administration of insulin or insulin secretagogues (sulfonylureas or meglitinide), and an increased level of insulin resistance due to the even higher plasma insulin levels can occur. The biguanides increase insulin sensitivity resulting in some correction of hyperglycemia.

However, the two biguanides, phenformin and metformin, can induce lactic acidosis and nausea/diarrhea. Metformin has fewer side effects than phenformin and is often prescribed for the treatment of type 2 diabetes.

The glitazones (i. e. 5-benzylthiazolidine-2,4-diones) are a newer class of compounds with the potential for ameliorating hyperglycemia and other symptoms of type 2 diabetes. These agents substantially increase insulin sensitivity in muscle, liver and adipose tissue in several animal models of type 2 diabetes, resulting in partial or complete correction of the elevated plasma levels of glucose without occurrence of hypoglycemia. The glitazones that are currently marketed are agonists of the peroxisome proliferator activated receptor (PPAR) gamma subtype. PPARgamma agonism is generally believed to be responsible for the improved insulin sensititization that is observed with the glitazones. Besides these positive effects glitazones promote adipocyte differentiation leading to undesirable weight gain. Newer PPAR agonists that are being developed for treatment of type 2 diabetes and/or dyslipidemia are agonists of one or more of the PPAR alpha, gamma and delta subtypes.

There is a continuing need for new methods of treating the disease.

New biochemical approaches that have been recently introduced or are under active development include treatment with alpha-glucosidase inhibitors (e. g. acarbose), protein tyrosine phosphatase-1B (PTP-1B) inhibitors, and inhibitors of the dipeptidyl peptidase-IV (DPP-IV) enzyme. Inhibition of the expression of PTP-1B by the use of antisense oligonucleotides is also under investigation.

Another method of treating type 2 diabetes that has been suggested in the literature is the use of inhibitors of the 11-β-hydroxysteroid dehydrogenase type 1 enzyme (11β-HSD 1) to reduce the amount of active glucocorticoids in tissues where glucose is metabolized.

Mono- and bicyclic thiazole sulfonamides are disclosed in WO-A 01/90090, WO-A 01/90091, WO-A 01/90092, WO-A 01/90093, WO-A 01/90094, WO-A 03/043999 and WO-A 2004/037251. Thiophene and thiadiazole sufonamides are known from WO-A 03/044009 and WO-A 03/044000. Additionally compounds containing a triazole moiety are known from WO-A 03/065983 and WO-A 03/104208.

Aryl and heteroaryl ketones are known from WO-A 04/011410. 1,4-Disubstituted piperidines are known from WO-A 04/033427 and 2-oxo-ethanesulfonamides are known from WO-A 04/041264. Adamantyl acetamides are known from WO-A 04/056744 and WO-A 04/056745. Amide derivatives are known from WO-A 04/065351.

Thus, the object of the present invention is to provide a new class of compounds as 11β-HSD 1 inhibitors which may be effective in the treatment of type 2 diabetes, metabolic syndrome and other 11 β-HSD 1 modulated diseases.

Accordingly, the present invention provides compounds of formula (I) 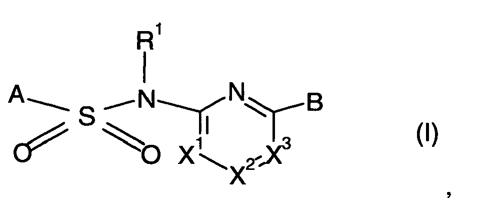 or a pharmaceutically acceptable salt, prodrug or metabolite thereof, wherein
X¹, X², X³ are independently selected from the group consisting of N; and CR², wherein R² is independently H; halogen; CN; C(O)OR³; C(O)N(R³R^{3a}); OR³; N(R³R^{3a}); C₁₋₆alkyl or C₃₋₇ cycloalkyl wherein C₁₋₆ alkyl and C₃₋₇ cycloalkyl are optionally substituted with one or more halogen, which are the same or different;
R³, R^{3a} are independently selected from the group consisting of H; C₁₋₆ alkyl; and C₃₋₇ cycloalkyl;
A is T; T-N(R^{1a})-; or C₁₋₆ alkyl, wherein C₁₋₆ alkyl is optionally substituted with one or more R⁵, wherein R⁵ is independently halogen; CN; COOR⁶; OR⁶; C(O)N(R⁶R^{6a}); S(O)₂N(R⁶R^{6a}); S(O)N(R⁶R^{6a}); S(O)₂R⁶; N(R⁶)S(O)₂N(R^{6a}N^{6b}); SR⁶; N(R⁶R^{6a}); OC(O)R⁶; N(R⁶)C(O)R^{6a}; N(R⁶)S(O)₂R^{6a}; N(R⁶)S(O)R^{6a}; N(R⁶)C(O)N(R^{6a}R^{6b}); N(R⁶)C(CO)OR^{6a}; OC(O)N(R⁶R^{6a}); or T;
R¹, R^{1a} are independently selected from the group consisting of H; allyl; benzyl; CH₂₋C(O)-O-C₁₋₆ alkyl; C₁₋₆ alkyl; and C₃₋₇ cycloalkyl, wherein C₁₋₆ alkyl or C₃₋₇ cycloalkyl is optionally substituted with one or more R⁴, wherein R⁴ is independently halogen; C₁₋₆ alkyl; or C₃₋₇ cycloalkyl;
R⁶, R^{6a}R^{6b} are independently selected from the group consisting of H; C₁₋₆ alkyl; and T, wherein C₁₋₆ alkyl is optionally substituted with one or more halogen, which are the same of different;
T is C₃₋₇ cycloalkyl; heterocyclyl; heterobicyclyl; phenyl; naphtyl; indenyl; indanyl; tetralinyl; decalinyl; or adamantyl, wherein T is optionally substituted with one or more R⁷, wherein R⁷ is independently halogen; CN; COOR⁸; OR⁸; C(O)N(R⁸R^{8a}); S(O)₂N(R⁸R^{8a}); S(O)N(R⁸R^{8a}); S(O)₂R⁸; N(R⁸)S(O)₂N(R^{8a}N^{8b}); SR⁸; N(R⁸R^{8a}); OC(O)R⁸; N(R⁸)C(O)R^{8a}; N(R⁸)S(O)₂R^{8a}; N(R⁸)S(O)R^{8a}; N(R⁸)C(O)N(R^{8a}R^{8b}); N(R⁸)C(O)OR^{8a}; OC(O)N(R⁸R^{8a}); oxo (=O), where the ring is at least partially saturated; C(O)R⁸; T¹; or C₁₋₆ alkyl, wherein C₁₋₆ alkyl is optionally substituted with one or more R⁹;
R⁸, R^{8a}, R^{8b} are independently selected from the group consisting of H; T¹; and C₁₋₆ alkyl, wherein C₁₋₆ alkyl is optionally substituted with one or more R¹⁰;
R⁹, R¹⁰ are independently selected from the group consisting of halogen; CN; COOR¹¹; OR¹¹; C(O)R¹¹; C(O)N(R¹¹R^{11a}); S(O)₂N(R¹¹R^{11a}); S(O)N(R¹¹R^{11a}); S(O)₂R¹¹; N(R¹¹)S(O)₂N(R^{11a}N^{11b}); SR¹¹; N(R¹¹R^{11a}); OC(O)R¹¹; N(R¹¹)C(O)R^{11a}; N(R¹¹)SO₂R^{11a}; N(R¹¹)S(O)R^{11a}; N(R¹¹)C(O)N(R^{11a}R^{11b}); N(R¹¹)C(O)OR^{11a}; OC(O)N(R¹¹R^{11a}); and T¹;
R¹¹, R^{11a}, R^{11b} are independently selected from the group consisting of H; C₁₋₆ alkyl; and T¹;
T¹ is C₃₋₇ cycloalkyl; heterocyclyl; heterobicyclyl; phenyl; naphtyl; indenyl; indanyl; tetralinyl; decalinyl; or adamantyl, wherein T¹ is optionally substituted with one or more R¹², wherein R¹² is independently halogen; CN; COOR¹³; OR¹³; C(O)N(R¹³R^{13a}); S(O)₂N(R¹³R^{13a}); S(O)N(R¹³R^{13a}); S(O)₂R¹³; N(R¹³)S(O)₂N(R^{13a}N^{13b}); SR¹³; N(R¹³R^{13a}); OC(O)R¹³; N(R¹³)C(O)R^{13a}; N(R¹³)S(O)₂R^{13a};N(R¹³)S(O)R^{13a}; N(R¹³)C(O)N(R^{13a}R^{13b}); N(R¹³)C(O)OR^{13a}; OC(O)N(R¹³R^{13a}); oxo (=O), where the ring is at least partially saturated; C(O)R¹³; C₁₋₆ alkyl; phenyl; C₃₋₇ cycloalkyl; or heterocyclyl, wherein C₁₋₆ alkyl; phenyl; C₃₋₇ cycloalkyl; and heterocyclyl are optionally substituted with one or more halogen, which are the same or different;
R¹³, R^{13a}, R^{13b} are independently selected from the group consisting of H; C₁₋₆alkyl; phenyl; C₃₋₇ cycloalkyl; and heterocyclyl, wherein C₁₋₆ alkyl; phenyl; C₃₋₇ cycloalkyl; and heterocyclyl are optionally substituted with one or more halogen, which are the same or different;
B is T²;
T² is C₃₋₇ cycloalkyl, heterocyclyl; heterobicyclyl; phenyl; naphtyl; indenyl; indanyl; tetralinyl; decalinyl; or adamantyl, wherein T² is optionally substituted with one or more R¹⁶;
R¹⁶ is independently selected from the group consisting of halogen; CN; COOR²²; OR²²; C(O)N(R²²R^{22a}); S(O)₂N(R²²R^{22a}); S(O)N(R²²R^{22a}); S(O)₂R²² ; N(R²²)S(O)₂N(R^{22a}N^{22b}); SR²²; N(R²²R^{22a}); OC(O)R²²; N(R²²)C(O)R^{22a}; N(R²²)S(O)2R^{22a}; N(R²²)S(O)R^{22a}; N(R²²)C(O)N(R^{22a}R^{22b}); N(R²²)C(O)OR^{22a}; OC(O)N(R²²R^{22a}); oxo (=O), where the ring is at least partially saturated; C(O)R²²; C₁₋₆ alkyl; phenyl; heterocyclyl; and C₃₋₇ cycloalkyl, wherein C₁₋₆ alkyl; phenyl; heterocyclyl; and C₃₋₇ cycloalkyl are optionally substituted with one or more R^{20a};
R²², R^{22a}, R^{22b} are independently selected from the group consisting of H; phenyl; heterocyclyl; C₃₋₇ cycloalkyl; and C₁₋₆ alkyl, wherein phenyl; heterocyclyl; C₃₋₇ cycloalkyl; and C₁₋₆ alkyl are optionally substituted with one or more R^{20b}
R^{20a}, R^{20b} are independently selected from the group consisting of halogen; CN; COOR²³; OR²³; C(O)R²³; C(O)N(R²³R^{23a}); S(O)₂N(R²³R^{23a}); S(O)N(R²³R^{23a}); S(O)₂R²³; N(R²³)S(O)₂N(R^{23a}N^{23b}); SR²³; N(R²³R^{23a}); OC(O)R²³; N(R²³)C(O)R^{23a}; N(R²³)SO₂R^{23a}; N(R²³)S(O)R^{23a} ; N(R²³)C(O)N(R^{23a}R^{23b}); N(R²³)C(O)OR^{23a}; OC(O)N(R²³R^{23a}); C₁₋₆ alkyl; phenyl; heterocyclyl; and C₃₋₇ cycloalkyl, wherein C₁₋₆ alkyl; phenyl; heterocyclyl; and C₃₋₇ cycloalkyl are optionally substituted with one or more R²⁴;
R²³ R^{23a}, R^{23b} are independently selected from the group consisting of H; phenyl; heterocyclyl; C₃₋₇ cycloalkyl and C₁₋₆ alkyl, wherein phenyl; heterocyclyl; C₃₋₇ cycloalkyl; and C₁₋₆ alkyl are optionally substituted with one or more R^{24a};
R²⁴, R^{24a} are independently selected from the group consisting of halogen; CN; COOR²⁵; OR²⁵; C(O)R²⁵; C(O)N(R²⁵R^{25a}); S(O)₂N(R²⁵R^{25a}); S(O)N(R²⁵R^{25a}); S(O)₂R²⁵; N(R²⁵)S(O)₂N(R^{25a}R^{25b}); SR²⁵; N(R²⁵R^{25a}); OC(O)R²⁵; N(R²⁵)C(O)R^{25a}; N(R²⁵)SO₂R^{25a}; N(R²⁵)S(O)R^{25a}; N(R²⁵)C(O)N(R^{25a}R^{25b}); N(R²⁵)C(O)OR^{25a}; OC(O)N(R²⁵R^{25a}); and C₁₋₆ alkyl, wherein C₁₋₆ alkyl is optionally substituted with one or more halogen, which are the same of different;
R²⁵, R^{25a}, R^{25b} are independently selected from the group consisting of H; C₁₋₆ alkyl, wherein C₁₋₆ alkyl is optionally substituted with one or more halogen, which are the same of different.

In case a variable or substituent can be selected from a group of different variants and such variable or substituent occurs more than once the respective variants can be the same or different.

Within the meaning of the present invention the terms are used as follows:

"Alkyl" means a straight-chain or branched carbon chain that may contain double or triple bonds. It is generally preferred that alkyl doesn't contain double or triple bonds. Each hydrogen of an alkyl carbon may be replaced by a substituent.

"C₁₋₄ alkyl" means an alkyl chain having 1 - 4 carbon atoms, e.g. if present at the end of a molecule: methyl, ethyl, -CH=CH₂, -C≡CH, n-propyl, isopropyl, -CH=CH-CH₃, -CH₂₋CH=CH₂, n-butyl, isobutyl, -CH=CH-CH₂-CH₃, -CH=CH-CH=CH₂, sec-butyl tert-butyl, or e.g. -CH₂-, -CH₂-CH₂-, -CH=CH-, -CH(CH₃)-, -C(CH₂)-, -CH₂-CH₂-CH₂-, -CH(C₂H₅)-, -CH(CH₃)₂-, when two moieties of a molecule are linked by the alkyl group. Each hydrogen of a C₁₋₄ alkyl carbon may be replaced by a substituent.

"C₁₋₆ alkyl" means an alkyl chain having 1 - 6 carbon atoms, e.g. if present at the end of a molecule: C₁₋₄ alkyl, methyl, ethyl, -CH=CH₂, -C≡CH, n-propyl, isopropyl, -CH=CH-CH₃, -CH₂-CH=CH₂, n-butyl, isobutyl, -CH=CH-CH₂-CH₃, -CH=CH-CH=CH₂, sec-butyl; tert-butyl, n-pentane, n-hexane, or e.g. -CH₂-, -CH₂-CH₂-, -CH=CH-, -CH(CH₃)-, -C(CH₂)-, - CH₂-CH₂-CH₂-, -CH(C₂H₅)-, -CH(CH₃)₂-, when two moieties of a molecule are linked by the alkyl group. Each hydrogen of a C₁₋₆ alkyl carbon may be replaced by a substituent.

"C₃₋₇ cycloalkyl" or "C₃₋₇ cycloalkyl ring" means a cyclic alkyl chain having 3 - 7 carbon atoms, e.g. cyclopropyl, cyclobutyl, cyclopentyl, cyclohexyl, cyclohexenyl, cycloheptyl. Each hydrogen of a cycloalkyl carbon may be replaced by a substituent.

"Halogen" means fluoro, chloro, bromo or iodo. It is generally preferred that halogen is fluoro or chloro.

"Heterocyclyl" or "heterocycle" means a cyclopentane, cyclohexane or cycloheptane ring that may contain up to the maximum number of double bonds (aromatic or non-aromatic ring which is fully, partially or un-saturated) wherein at least one carbon atom up to 4 carbon atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for a heterocycle are furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, azepine or homopiperazine. "Heterocycle" means also azetidine.

"Heterobicyclyl" or "heterobicycle" means a heterocycle which is condensed with phenyl, C₃₋₇ cycloalkyl or an additional heterocycle to form a bicyclic ring system. "Condensed" to form a bicyclic ring means that two rings are attached to each other by sharing two ring atoms. Examples for a heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, quinoline, dihydroquinoline, tetrahydroquinoline, decahydroquinoline, isoquinoline, decahydroisoquinoline, tetrahydroisoquinoline, dihydroisoquinoline, benzazepine, purine or pteridine.

Preferred compounds of formula (I) are those compounds in which one or more of the residues contained therein have the meanings given below, with all combinations of preferred substituent definitions being a subject of the present invention. With respect to all preferred compounds of the formulas (I) the present invention also includes all tautomeric and stereoisomeric forms and mixtures thereof in all ratios, and their pharmaceutically acceptable salts.

In preferred embodiments of the present invention, the substituents A, B, R¹ and X¹ to X³ of the formula (I) independently have the following meaning. Hence, one or more of the substituents A, B, R¹ and X¹ to X³ can have the preferred or more preferred meanings given below.

Preferably, at most two of X¹, X² and X³ are N.

Preferably, R² is H.

Preferably, R¹ is H.

Preferably, A is phenyl, C₃₋₇ cycloalkyl; heterocyclyl; or C₁₋₆ alkyl. More preferred A is phenyl; cyclohexyl; thiophenyl; diazolyl; pyridyl; ethyl; isopropyl; n-butyl; or n-pentyl.

It is also preferred that A is T and T is heterobicyclyl, more preferred T is decahydroquinoline or tetrahydroisoquinoline, wherein the ring nitrogen is directly linked to the sulphur of the sulphonamide group of formula (I).

Preferably, A is T or T-NH- and T is optionally substituted with up to 3R⁷, independently being F; Cl; CH₃; OCH₃; N(CH₃)₂; T¹; OT¹; or NHT¹. More preferred T¹ is phenyl, which is optionally substituted with up to three halogens, independently being F; or Cl.

Preferably, T² is phenyl, which is unsubstituted or substituted with up to three R¹⁶, which are the same or different. Preferably, R¹⁶ is independently selected from the group consisting of F, Cl, COOR ²², C(O)NR²²R^{22a}, NR²²R^{22a} and CF₃.

Compounds of the formula (I) in which some or all of the above-mentioned groups have the preferred or more preferred meanings are also an object of the present invention.

A compound according to the present invention is preferred, wherein A is 3-chloro-2-methylphenyl; R¹ is H; X¹, X² and X³ are -CH= and B is phenyl.

Prodrugs of the compounds of the invention are also within the scope of the present invention.

"Prodrug" means a derivative that is converted into a compound according to the present invention by a reaction with an enzyme, gastric acid or the like under a physiological condition in the living body, e.g. by oxidation, reduction, hydrolysis or the like, each of which is carried out enzymatically. Examples of a prodrug are compounds, wherein the amino group in a compound of the present invention is acylated, alkylated or phosphorylated to form, e.g., eicosanoylamino, alanylamino, pivaloyloxymethylamino or wherein the hydroxyl group is acylated, alkylated, phosphorylated or converted into the borate, e.g. acetyloxy, palmitoyloxy, pivaloyloxy, succinyloxy, fumaryloxy, alanyloxy or wherein the carboxyl group is esterified or amidated. These compounds can be produced from compounds of the present invention according to well-known methods.

Metabolites of compounds of formula (I) are also within the scope of the present invention.

Where tautomerism, like e.g. keto-enol tautomerism, of compounds of general formula (I) may occur, the individual forms, like e.g. the keto and enol form, are comprised separately and together as mixtures in any ratio. Same applies for stereoisomers, like e.g. enantiomers, cis/trans isomers, conformers and the like.

If desired, isomers can be separated by methods well known in the art, e.g. by liquid chromatography. Same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of formula (I) may be obtained from stereoselective synthesis using optically pure starting materials.

In case the compounds according to formula (I) contain one or more acidic or basic groups, the invention also comprises their corresponding pharmaceutically or toxicologically acceptable salts, in particular their pharmaceutically utilizable salts. Thus, the compounds of the formula (I) which contain acidic groups can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I) simultaneously contain acidic and basic groups in the molecule, the invention also includes, in addition to the salt forms mentioned, inner salts or betaines (zwitterions). The respective salts according to the formula (I) can be obtained by customary methods which are known to the person skilled in the art like, for example by contacting these with an organic or inorganic acid or base in a solvent or dispersant, or by anion exchange or cation exchange with other salts. The present invention also includes all salts of the compounds of the formula (I) which, owing to low physiological compatibility, are not directly suitable for use in pharmaceuticals but which can be used, for example, as intermediates for chemical reactions or for the preparation of pharmaceutically acceptable salts.

The present invention provides compounds of general formula (I) as 11β-HSD1 inhibitors. The biochemical mechanism, where the 11β-HSD1 enzyme may play a role is described in more detail in WO-A 03/065983 under the paragraph "Biochemical Mechanism" starting on page 18, which paragraph is herewith incorporated by reference.

Accordingly, the tissue specific 11β-HSD1 enzyme and its counterpart 11β-HSD2 may play a role for the modulation of glucocorticoid concentrations. Glucocorticoids (also called corticosteroids) are steroid hormones that play an important role in mammals, including humans. Control or modulation of glucocorticoid activity is important in regulating physiological processes in a wide range of tissues and organs. 11 β-HSD 1 and 11β-HSD2 have different cofactor requirements and substrate affinities. The 11β-hydroxysteroid dehydrogenase type 2 enzyme (1 1β-HSD2) is a high affinity enzyme that generally uses NAD⁺ as the preferred cofactor and rapidly dehydrogenates 11β-hydroxy-glucocorticoids, such as cortisol, to 11-keto glucocorticoids, such as cortisone. The 11β-hydroxysteroid dehydrogenase type 1 enzyme (11β-HSD1) is a low affinity enzyme that generally uses NADP⁺/NADPH as a cofactor rather than NAD⁺ (Agarwal et al., 1994, J. Biol. Chem., 269: 25959-25962). In vitro studies have shown that 11β-HSD1 is capable of acting as both a reductase and a dehydrogenase. However, 11β-HSD1 in vivo mainly acts as a reductase, converting 11-ketoglucocorticoids, such as cortisone, to 11β-hydroxyglucocorticoids, such as cortisol. Therefore 11β-HSD1 regulates the intracellular pool of the insulin antagonists 11β-hydroxyglucocorticoids, such as cortisol in humans, especially in metabolically active tissues such as liver and adipose tissue. Hence, increased 11β-HSD1 activity might be a cause for the development of type 2 diabetes mellitus, metabolic syndrome and related diseases, e. g. obesity, cardiovascular disease and hypertension.

11β-HSD1 enzyme related or modulated diseases are also described in more detail in WO-A 03/065983 under the paragraph "Utilities" starting on page 20, which paragraph is herewith incorporated by reference.

Another disease related to the 11-HSD1 enzyme is Alzheimer's disease and its inherent mild cognitive impairment (MCI) that is a common feature of aging but is, at present, untreatable (Ritchie, K. & Touchon, J. 2000). MCI and Alzheimer's disease are associated with a poor quality of life and loss of independence in older people and results in huge socioeconomic costs (Fillit, H. et al. 2002). In aged animals and humans, interindividual differences in cognitive function have been ascribed to variations in long-term glucocorticoid exposure (Lupien, S. J. et al. 1998; Seckl, J. R. & Olsson, T. 1995). The hippocampus plays a central role in the formation of long-lasting memories, highly expresses receptors for glucocorticoids in rodents (Reul, J. M. H. M. & de Kloet, E. R. 1985) and humans (Seckl, J. R. et al. 1991), and is particularly sensitive to the deleterious actions of chronic glucocorticoid excess (Landfield, P. W. et al. 1978; Meaney, M. J. et al. 1995). Dysregulation of the hypothalamic-pituitary-adrenal (HPA) axis with resultant chronically increased exposure of the hippocampus to elevated glucocorticoid levels has been hypothesized to contribute to the decline in cognitive function with aging, through the detrimental effects of glucocorticoids on hippocampal neurons (McEwen, B. S. & Sapolsky, R. M. 1995; McEwen, B. S. 1999). In human populations, including those with Alzheimer's disease and normal aging, higher cortisol levels have been associated with poorer memory and hippocampal shrinkage/neuronal loss (Lupien, S. J. et al. 1998; Newcomer, J. W. et al. 1994; de Leon, M. J. et al. 1988). 11β-HSDs are also expressed in the adult rat brain (Moisan, M.-P. et al. 1990; Lakshmi, V. et al. 1991; Sakai, R. R. et al. 1992) where 11β-HSD1 is abundant, but 11β-HSD2 is largely absent (Roland, B. L. et al. 1995). In intact rat hippocampal cells in primary culture, which express solely the 11β-HSD1 isozyme, the reaction catalyzed is reduction. Such reactivation of glucocorticoids by 11β-HSD1 allows intrinsically inert 11-keto steroids to mimic active glucocorticoids in potentiating kainate-induced neurotoxicity (Rajan, V. et al. 1996). Carbenoxolone, which inhibits unspecifically both isozymes of 11β-HSD in vitro and in vivo (Stewart, P. M. et al. 1990), prevents regeneration of glucocorticoids by 11β-HSD1 and thereby protects primary cultures of hippocampal cells from glucocorticoid-mediated exacerbation of excitatory amino acid neurotoxicity (Rajan, V. et al. 1996). Regeneration of glucocorticoids by 11β-HSD1 within neurons seems important in vivo because 11β-HSD1 knockout mice are protected from glucocorticoid-associated hippocampal dysfunction with aging (Yau, J. L. W. et al. 2001). Therefore specific inhibition of 11β-HSD 1 might additionally protect against age-dependent cognitive impairment. Furthermore, administration of the 11β-HSD inhibitor carbenoxolone improved verbal fluency in healthy elderly men and improved verbal memory in patients with type 2 diabetes (Sandeep T. C. et al. 2004).

Highly active antiretroviral therapy (HAART) in patients infected with human immunodeficiency virus (HIV) is associated with a poorly understood lipodystrophic and hypertriglyceridaemic syndrome, which resembles Cushing's syndrome, but in which plasma cortisol is not elevated. In adipose tissue of these patients with HAART-associated lipodystrophy, 11β-HSD 1 mRNA is increased and its concentration is correlated with features of insulin resistance. Therefore highly active antiretroviral therapy (HAART)-associated lipodystrophy might be explained by increased local regeneration of cortisol from inactive cortisone within adipose tissue, catalyzed by the enzyme 11β-HSD1 (Sutinen J. et al., Diabetologia Epub 29.09.2004).

Accordingly, the present invention provides compounds of formula (I) or pharmaceutically acceptable salts thereof for use as a medicament.

Furthermore, the present invention provides the use of a compound of formula (I) or a pharmaceutically acceptable salt thereof for the manufacture of a medicament for the treatment or prophylaxis of non-insulin dependent diabetes mellitus, hyperglycemia, low glucose tolerance, insulin resistance, β-cell dysfunction, obesity, lipid disorders, dyslipidemia, hyperlipidemia, hypertriglyceridemia, hypercholesterolemia, low HDL levels, high LDL levels, atherosclerosis and its sequelae, vascular restenosis, pancreatitis, abdominal obesity, neurodegenerative disease, retinopathy, nephropathy, neuropathy, Syndrome X, Alzheimer's disease, HAART-associated lipodystrophy or other conditions and disorders where insulin resistance is a component or that may be treated by inhibition of the 11β-HSD1 enzyme.

The present invention also provides a method for treating, controlling, delaying or preventing in a mammalian patient in need of treatment one or more conditions selected from the group consisting of the onset of non-insulin dependent diabetes mellitus, hyperglycemia, low glucose tolerance, insulin resistance, β-cell dysfunction, obesity, lipid disorders, dyslipidemia, hyperlipidemia, hypertriglyceridemia, hypercholesterolemia, low HDL levels, high LDL levels, atherosclerosis and its sequelae, vascular restenosis, pancreatitis, abdominal obesity, neurodegenerative disease, retinopathy, nephropathy, neuropathy, Syndrome X, Alzheimer's disease, HAART-associated lipodystrophy and other conditions and disorders where insulin resistance is a component or that may be treated by inhibition of the 11β-HSD1 enzyme, wherein the method comprises the administration to said patient of a therapeutically effective amount of a compound according to the present invention or a pharmaceutically acceptable salt thereof.

The present invention provides pharmaceutical compositions comprising a compound of formula (I) or a pharmaceutically acceptable salt thereof as active ingredient together with a pharmaceutically acceptable carrier, optionally in combination with one or more other pharmaceutical compositions.

"Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier.

A pharmaceutical composition of the present invention may comprise one or more additional compounds as active ingredients like one or more compounds of formula (I) not being the first compound in the composition or other 11β-HSD1 inhibitors.

Other active ingredients are disclosed, e.g., in WO-A 03/065983 under the paragraph "Combination Therapy" starting on page 27, which paragraph is herewith incorporated by reference.

Accordingly, other active ingredients may be DPP-IV inhibitors; insulin sensitizers selected from the group consisting of PPAR agonists and biguanides; insulin and insulin mimetics; sulfonylureas and other insulin secretagogues; α-glucosidase inhibitors; glucagon receptor antagonists; GLP-1, GLP-1 mimetics, and GLP-1 receptor agonists; GIP, GIP mimetics, and GIP receptor agonists; PACAP, PACAP mimetics, and PACAP receptor 3 agonists; cholesterol lowering agents selected from the group consisting of HMG-CoA reductase inhibitors, sequestrants, nicotinyl alcohol, nicotinic acid or a salt thereof, PPARγ agonists, PPARα/γ dual agonists, inhibitors of cholesterol absorption, acyl CoA: cholesterol acyltransferase inhibitors, and anti-oxidants; PPAR agonists; antiobesity compounds ; ileal bile acid transporter inhibitors; anti-inflammatory agents; or protein tyrosine phosphatase-1B (PTP-1B) inhibitors.

Other active ingredients may be anti-hypertensive compounds or compounds for the treatment of cardiovascular diseases, particularly atherosclerosis.

The active ingredients may be comprised in one or more different pharmaceutical compositions (combination of pharmaceutical compositions).

The term "pharmaceutically acceptable salts" refers to salts prepared from pharmaceutically acceptable non-toxic bases or acids, including inorganic bases or acids and organic bases or acids.

The compositions include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, the compounds of formula (I) can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally, for example, as liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds of formula (I) may also be administered parenterally. Solutions or suspensions of these active compounds can be prepared in water suitably mixed with a surfactant such as hydroxypropyl-cellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of formula (I) are administered orally.

The effective dosage of active ingredient employed may vary depending on the particular compound employed, the mode of administration, the condition being treated and the severity of the condition being treated. Such dosage may be ascertained readily by a person skilled in the art.

When treating or preventing diabetes mellitus and/or hyperglycemia or hypertriglyceridemia or other diseases for which compounds of formula (I) are indicated, generally satisfactory results are obtained when the compounds of the present invention are administered at a daily dosage of from about 0.1 milligram to about 100 milligram per kilogram of animal body weight, preferably given as a single daily dose or in divided doses two to six times a day, or in sustained release form. For most large mammals, the total daily dosage is from about 1.0 milligrams to about 1000 milligrams, preferably from about 1 milligram to about 50 milligrams. In the case of a 70 kg adult human, the total daily dose will generally be from about 7 milligrams to about 350 milligrams. This dosage regimen may be adjusted to provide the optimal therapeutic response.

Some abbreviations that may appear in this application are as follows.

### Abbreviations

### Designation

- Boc: ^{*t*}Butoxycarbonyl
- Boc₂O: Di-*tert*-butyl-dicarbonate
- *n*BuLi: *n*-Butyl lithium
- DCM: Dichloromethane
- DMAP: Dimethylaminopyridine
- DME: Dimethoxyethane
- DMF: *N,N*-Dimethylformamide
- EDC: 1-(3-Dimethylaminopropyl)-3-ethylcarbodiimide hydrochloride
- EtOH: Ethanol
- HOBt: 1-Hydroxybenzotriazole
- LDA: Lithium diisopropylamine
- mCPBA: *meta*-Chloroperbenzoic acid
- MeCN: Acetonitrile
- MeOH: Methanol
- TBTU: 2-(1H-Benzotriazole-1-yl)-1,1,3,3-tetramethyluronium tetrafluoroborate
- THF: Tetrahydrofuran

Available starting materials for the synthesis of preferred embodiments of the invention may be amines of formula (II) and sulfonyl chlorides of the formula (III). They may be purchased from commercially available sources such as Array, Sigma Aldrich, Fluka, ABCR or be synthesized by one skilled in the art.

In general compounds of the formula (I) 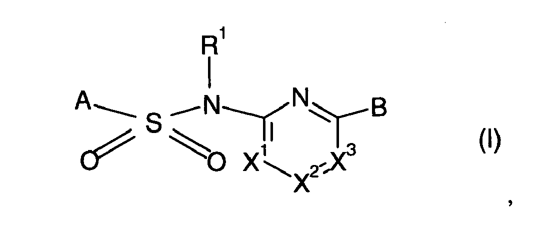 wherein the variables have the above described meanings (unless otherwise specifically indicated) may be prepared by coupling a compound of formula (II) 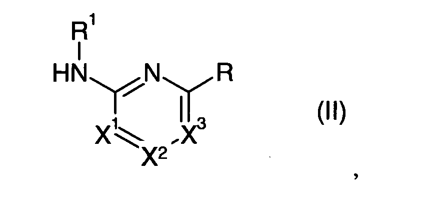 wherein R is T²
with a compound of the formula A-S(O)₂-Cl, wherein A is T or C₁₋₆ alkyl; or
with a compound of the formula A-H and SO₂Cl₂, wherein A is T-N(R^{1a})-.

More specific routes to prepare preferred embodiments of compounds of the present invention are outlined in schemes A and B.

Sulfonamides of the present invention may be synthesised by initial formation of the sulfonamide moiety followed by the introduction of aryl/heteroaryl T² using Suzuki chemistry.

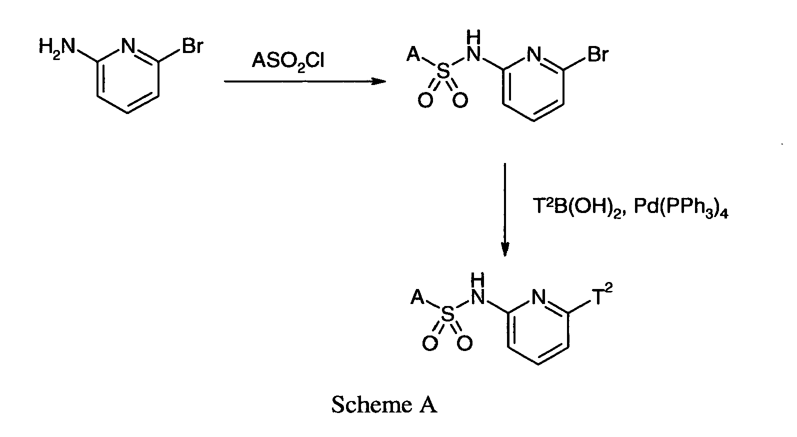

The sulfamide moiety in scheme B can be introduced using a literature procedure (J. Med. Chem., 1972, 15, 5, 538).

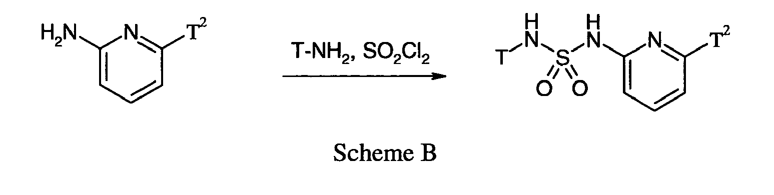

Compounds of the present invention may also be synthesised according to scheme C

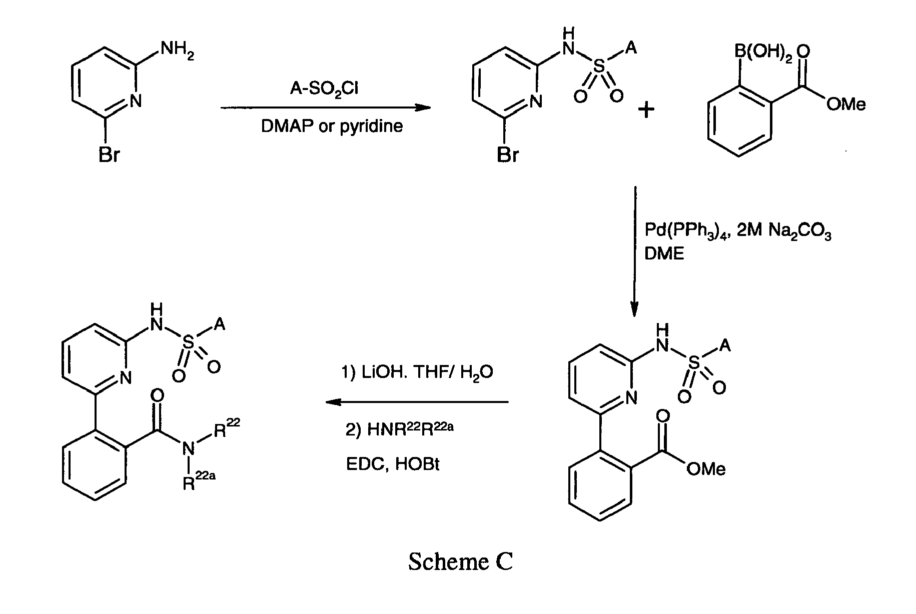

### Examples

### 11β-HSD Assays

### Biochemical assays monitoring the activity of mouse 11β-HSD type 1, human 11β-HSD type 1 and type 2

As source for the respective enzyme the human embryonic kidney cell line, HEK 293, was transfected with the full length cDNAs for human and mouse 11β-HSD1 and human 11β-HSD2 followed by selection of stably expressing clones according to standard procedures.

Whole cell lysates of 11β-HSD cell lines were prepared by growing of cells in 15 cm dishes in culture medium (DMEM, 10% FCS and 600 µg/ml G418) until 70% confluency. To force expression of 11β-HSD, cells were incubated overnight with 1.5 mM sodium butyrate (Aldrich, cat. no. 30,341-0), a histone deacetylase inhibitor. Cells were then harvested by addition of 2 ml PBS with 2.5 mM EDTA per 15 cm dish for 5 to 10 min at RT. Cell suspensions were combined and after centrifugation by 1000g for 5 min the cell pellet was homogenized in 20 mM potassium phosphate buffer, pH 7.4, 5 mM EDTA, 250 mM sucrose (1.5 ml per cell pellet of five 15 cm dishes) using a Ultra Turrax Homogenizer. The whole cell lysate was frozen in liquid nitrogen and stored at -80°C in aliquots suitable for single use.

The enzymatic reaction was carried out in the presence of cofactors NAD⁺ (11B-HSD2) or NADPH (11β-HSD1) and the respective steroid substrates. In order to maintain an excess of the cofactor NADPH, NADPH was regenerated by adding glucose-6-phosphate (G6P) and G6P-dehydrogenase in non-limiting concentrations to the 11β-HSD1 enzymatic reaction. All assay components and compounds were diluted in reaction buffer, 20 mM Tris-HCl (pH 7.4), 250 mM sucrose, 100 mM NaCl, 1 mM MgCl2, 1 mM EDTA, 1 mM EGTA, and stored on ice prior to use.

The following reaction mixtures plus compounds in concentrations between 10 pM and 300 µM were prepared in a total volume of 102 µl and incubated for 15 min at 37°C.

Human and mouse 11β-HSD type 1 reductase activity:
100 µM NADPH (AppliChem, cat. no. A1395,0500), 10 mM glucose-6-phosphate (Sigma cat. no. G-7879), 0.37 U/ml glucose-6-phosphate-dehydrogenase (Fluka, cat. no 49275) and 3 µM 11-dehydrocorticosterone (Research Plus, cat. no. 3306-16) or 3 µM cortisone (Sigma, cat. no. C-2755) for mouse and human enzyme, respectively.
The compounds exemplified in this invention typically have IC₅₀ values < 100 µM

Human 11β-HSD type 2 hydrogenase activity:
100 µM NAD⁺ (Applichem, cat. no. A1124,0001) and 3 µM cortisol (Sigma, cat. no H-0888).

The enzymatic reaction was started by adding 18 µl whole cell lysates, equivalent to 80.000 cells of the respective HSD overexpressing cell lines. After 60 minutes at 37°C the reaction was stopped by carbenoxolone (Sigma, cat. no C-4790), an inhibitor of all HSD enzymes analysed, in a final concentration of 100 µM.

To determine the extent of the enzymatic reactions, cortisole or corticosterone were measured in an ELISA-based assay principle. This cortisol/corticosterone ELISA makes use of a monoclonal antibody specifically binding to cortisol/corticosterone. The detection is competition-based, since cortisol or corticosterone in a sample compete with cortisol covalently attached to alkaline phosphatase (AP). The amount of alkaline phosphatase bound, was detected by a colourimetric measurement, using pNPP as phosphatase substrate.

96well ELISA plates (Falcon, cat. no. BD 353915) were coated with 5 µg/ml rabbit anti mouse IgG antibody (Sigma, cat. no. M-9637) in PBS overnight at 4°C. After aspiration of this solution the wells were blocked with 200 mM acetate citrate buffer, pH 6.0, 20% FCS and 2% gelatine for one hour at RT. The plates were washed three times (50 mM Tris-HCl, pH 7.8, 0.1% Tween 20) and 100 µl of samples and standards, cortisol or corticosterone ranging from 78 nM to 10 µM, were added. The stocks of Cortisol-AP conjugate (Fitzgerald, cat. no. 65-IC07) and mouse monoclonal anti-cortisol antibody (Biotrend cat. no. 2330-4879) were diluted 1:1600 or 1:3600, respectively, in 100 mM Tris-HCl, pH 7.7, 10 mM MgCl₂, 0.1% BSA. 50 µl of each dilution were added per well. After a incubation of two hours at RT under shaking, plates were washed three times (50 mM Tris-HCl, pH 7.8,0.1% Tween 20).

For detection, 200 µl of 25 mM Glycin, pH 10.3, 10 mM pNPP (Fluka, cat. no. 71768), 1 mM ZnSO₄ and 1 mM MgSO₄, were added and incubated for two hours at RT under shaking. Colourimetric measurement was performed in a microplate reader at 405 nm (Versamax, Molecular Devices). The concentrations of cortisol or corticosterone were calculated with SoftmaxPro software (Molecular devices) relative to standard values. The inhibitory potency of 11β-HSD inhibitors was determined by IC50 calculation with Prism 4.0 software (GraphPad).

The compounds of the present invention may show no activity against the 11β-HSD-2 enzyme.

### Cell-based human 11β-HSD type 1 assay

30.000 11β-HSD1 stably expressing HEK293 cells were seeded per 96 well in 100 µl culture medium (DMEM, 10% FCS and 600 µg/ml G418). After overnight incubation at 37°C and 5% CO₂ 11β-HSD inhibitors in final concentrations between 10 pM and 300 µM, 3 µM cortisone and 20 mM HEPES were added to the culture medium. All components, i.e. inhibitors, steroid substrate and HEPES were pre-diluted in DMEM prior to use. After incubation of 4 h at 37°C the culture medium was diluted 1:10 in 100 mM Tris-HCl, pH 7.7, 10 mM MgCl₂ and 0.1% BSA. The determination of cortisol concentrations was carried out with a commercially available Cortisol ELISA (Assay Designs, cat. no. 901071) according to the recommended protocol. The concentrations of cortisol were calculated with SoftmaxPro software (Molecular devices), the inhibitory potency of 11β-HSD inhibitors was determined by IC50 calculation with Prism 4.0 software (GraphPad).

The compounds according to the present invention typically may have IC₅₀ values < 100 µM.

## Claims

1. A sulfonamide compound of formula (I) 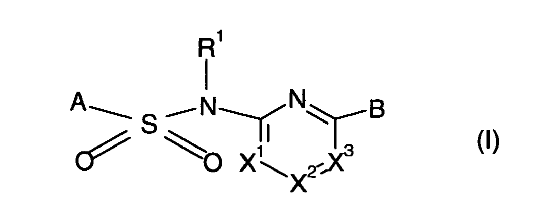 or a pharmaceutically acceptable salt, prodrug or metabolite thereof, wherein
X¹, X², X³ are independently selected from the group consisting of N; and CR², wherein R² is independently H; halogen; CN; C(O)OR³; C(O)N(R³R^{3a}); OR³; N(R³R^{3a}); C₁₋₆ alkyl or C₃₋₇ cycloalkyl wherein C₁₋₆ alkyl and C₃₋₇ cycloalkyl are optionally substituted with one or more halogen, which are the same or different;
R³, R^{3a} are independently selected from the group consisting of H; C₁₋₆alkyl; and C₃₋₇ cycloalkyl;
A is T; T-N(R^{1a})- or C₁₋₆ alkyl, wherein C₁₋₆ alkyl is optionally substituted with one or more R⁵, wherein R⁵ is independently halogen; CN; COOR⁶; OR⁶; C(O)N(R⁶R^{6a}); S(O)₂N(R⁶R^{6a}); S(O)N(R⁶R^{6a}); S(O)₂R⁶; N(R⁶)S(O)₂N(R^{6a}N^{6b}); SR⁶; N(R⁶R^{6a}); OC(O)R⁶; N(R⁶)C(O)R^{6a}; N(R⁶)S(O)₂R^{6a}; N(R⁶)S(O)R^{6a}; N(R⁶)C(O)N(R^{6a}R^{6b}); N(R⁶)C(CO)OR^{6a}; OC(O)N(R⁶R^{6a}); or T;
R¹, R^{1a} are independently selected from the group consisting of H; allyl; benzyl; CH₂-C(O)-O-C₁₋₆ alkyl; C₁₋₆ alkyl; and C₃₋₇ cycloalkyl, wherein C₁₋₆ alkyl or C₃₋₇ cycloalkyl is optionally substituted with one or more R⁴, wherein R⁴ is independently halogen; C₁₋₆ alkyl; or C₃₋₇ cycloalkyl;
R⁶, R^{6a} R^{6b} are independently selected from the group consisting of H; C₁₋₆ alkyl; and T, wherein C₁₋₆ alkyl is optionally substituted with one or more halogen, which are the same of different;
T is C₃₋₇ cycloalkyl; heterocyclyl; heterobicyclyl; phenyl; naphtyl; indenyl; indanyl; tetralinyl; decalinyl; or adamantyl, wherein T is optionally substituted with one or more R⁷, wherein R⁷ is independently halogen; CN; COOR⁸; OR⁸; C(O)N(R⁸R^{8a}); S(O)₂N(R⁸R^{8a}); S(O)N(R⁸R^{8a}); S(O)₂R⁸; N(R⁸)S(O)₂N(R^{8a}N^{8b}); SR⁸; N(R⁸R^{8a}); OC(O)R⁸; N(R⁸)C(O)R^{8a}; N(R⁸)S(O)₂R^{8a}; N(R⁸)S(O)R^{8a}; N(R⁸)C(O)N(R^{8a}R^{8b}); N(R⁸)C(O)OR^{8a}; OC(O)N(R⁸R^{8a}); oxo (=O), where the ring is at least partially saturated; C(O)R⁸; T¹; or C₁₋₆ alkyl, wherein C₁₋₆ alkyl is optionally substituted with one or more R⁹;
R⁸, R^{8a}, R^{8b} are independently selected from the group consisting of H; T¹; and C₁₋₆ alkyl, wherein C₁₋₆ alkyl is optionally substituted with one or more R¹⁰;
R⁹, R¹⁰ are independently selected from the group consisting of halogen; CN; COOR¹¹; OR¹¹; C(O)R¹¹; C(O)N(R¹¹R^{11a}); S(O)₂N(R¹¹R^{11a}); S(O)N(R¹¹R^{11a}); S(O)₂R¹¹; N(R¹¹)S(O)₂N(R^{11a}N^{11b}); SR¹¹; N(R¹¹R^{11a}); OC(O)R¹¹; N(R¹¹)C(O)R^{11a}; N(R¹¹)SO₂R^{11a}; N(R¹¹)S(O)R^{11a}; N(R¹¹)C(O)N(R^{11a}R^{11b}); N(R¹¹)C(O)OR^{11a}; OC(O)N(R¹¹R^{11a}); and T¹;
R¹¹, R^{11a}, R^{11b} are independently selected from the group consisting of H; C₁₋₆ alkyl; and T¹;
T¹ is C₃₋₇ cycloalkyl; heterocyclyl; heterobicyclyl; phenyl; naphtyl; indenyl; indanyl; tetralinyl; decalinyl; or adamantyl, wherein T¹ is optionally substituted with one or more R¹², wherein R¹² is independently halogen; CN; COOR¹³; OR¹³; C(O)N(R¹³R^{13a}); S(O)₂N(R¹³R^{13a}); S(O)N(R¹³R^{13a}); S(O)₂R¹³; N(R¹³)S(O)₂N(R^{13a}N^{13b}); SR¹³; N(R¹³R^{13a}); OC(O)R¹³; N(R¹³)C(O)R^{13a}; N(R¹³)S(O)₂R^{13a};N(R¹³)S(O)R^{13a}; N(R¹³)C(O)N(R^{13a}R^{13b}); N(R¹³)C(O)OR^{13a}; OC(O)N(R¹³R^{13a}); oxo (=O), where the ring is at least partially saturated; C(O)R¹³; C₁₋₆ alkyl; phenyl; C₃₋₇ cycloalkyl; or heterocyclyl, wherein C₁₋₆ alkyl; phenyl; C₃₋₇ cycloalkyl; and heterocyclyl are optionally substituted with one or more halogen, which are the same or different;
R¹³, R^{13a}, R^{13b} are independently selected from the group consisting of H; C₁₋₆ alkyl; phenyl; C₃₋₇ cycloalkyl; and heterocyclyl, wherein C₁₋₆ alkyl; phenyl; C₃₋₇ cycloalkyl; and heterocyclyl are optionally substituted with one or more halogen, which are the same or different;
B is T²;
T² is C₃₋₇ cycloalkyl, heterocyclyl; heterobicyclyl; phenyl; naphtyl; indenyl; indanyl; tetralinyl; decalinyl; or adamantyl, wherein T² is optionally substituted with one or more R¹⁶;
R¹⁶ is independently selected from the group consisting of halogen; CN; COOR²²; OR²²; C(O)N(R²²R²¹); S(O)₂N(R²²R^{22a}); S(O)N(R²²R^{22a}); S(O)₂R²²; N(R²²)S(O)₂N(R^{22a}N^{22b}); SR²²; N(R²²R^{22a}); OC(O)R²²; N(R²²)C(O)R^{22a}; N(R²²)S(O)₂R^{22a}; N(R²²)S(O)R^{22a}; N(R²²)C(O)N(R^{22a}R^{22b}); N(R²²)C(O)OR^{22a}; OC(O)N(R²²R^{22a}); oxo (=O), where the ring is at least partially saturated; C(O)R²²; C₁₋₆ alkyl; phenyl; heterocyclyl; and C₃₋₇ cycloalkyl, wherein C₁₋₆ alkyl; phenyl; heterocyclyl; and C₃₋₇ cycloalkyl are optionally substituted with one or more R^{20a};
R²², R^{22a}, R^{22b} are independently selected from the group consisting of H; phenyl; heterocyclyl; C₃₋₇ cycloalkyl; and C₁₋₆ alkyl, wherein phenyl; heterocyclyl; C₃₋₇ cycloalkyl; and C₁₋₆ alkyl are optionally substituted with one or more R^{20b}
R^{20a}, R^{20b} are independently selected from the group consisting of halogen; CN; COOR²³; OR²³; C(O)R²³; C(O)N(R²³R^{23a}); S(O)₂N(R²³R^{23a}); S(O)N(R²³R^{23a}); s(O)₂R²³; N(R²³)S(O)₂N(R^{23a}N^{23b}); SR²³; N(R²³R^{23a}); OC(O)R²³; N(R²³)C(O)R^{23a}; N(R²³)SO₂R^{23a}; N(R²³)S(O)R^{23a}; N(R²³)C(O)N(R^{23a}R^{23b}); N(R²¹)C(O)OR^{23a}; OC(O)N(R²³R^{23a}); C₁₋₆ alkyl; phenyl; heterocyclyl; and C₃₋₇ cycloalkyl, wherein C₁₋₆ alkyl; phenyl; heterocyclyl; and C₃₋₇ cycloalkyl are optionally substituted with one or more R²⁴;
R²³, R^{23a}, R^{23b} are independently selected from the group consisting of H; phenyl; heterocyclyl; C₃₋₇ cycloalkyl and C₁₋₆ alkyl, wherein phenyl; heterocyclyl; C₃₋₇ cycloalkyl; and C₁₋₆ alkyl are optionally substituted with one or more R^{24a};
R²⁴, R^{24a} are independently selected from the group consisting of halogen; CN; COOR²⁵; OR²⁵; C(O)R²⁵; C(O)N(R²⁵R^{25a}); S(O)₂N(R²⁵R^{25a}); S(O)N(R²⁵R^{25a}); S(O)₂R²⁵; N(R²⁵)S(O)₂N(R^{25a}R^{25b}); SR²⁵; N(R²⁵R^{25a}); OC(O)R²⁵; N(R²⁵)C(O)R^{25a}; N(R²⁵)SO₂R^{25a}; N(R²⁵)S(O)R^{25a}; N(R²⁵)C(O)N(R^{25a}R^{25b}); N(R²⁵)C(O)OR^{25a}; OC(O)N(R²⁵R^{25a}); and C₁₋₆ alkyl, wherein C₁₋₆ alkyl is optionally substituted with one or more halogen, which are the same of different;
R²⁵, R^{25a}, R^{25b} are independently selected from the group consisting of H; C₁₋₆ alkyl, wherein C₁₋₆ alkyl is optionally substituted with one or more halogen, which are the same of different.

2. A compound according to claim 1, wherein at most two of X¹, X² and X³ are N.

3. A compound according to claim 1 or 2, wherein R² is H.

4. A compound according to any of claims 1 to 3, wherein R¹ is H.

5. A compound according to any of claims 1 to 4, wherein A is phenyl, C₃₋₇ cycloalkyl; heterocyclyl; or C₁₋₆ alkyl.

6. A compound according to claim 5, wherein A is phenyl; cyclohexyl; thiophenyl; diazolyl; pyridyl; ethyl; isopropyl; n-butyl; or n-pentyl.

7. A compound according to any of claims 1 to 4, wherein A is T and T is heterobicyclyl.

8. A compound according to claim 7, wherein heterobicyclyl is decahydroquinoline or tetrahydroisoquinoline and wherein the ring nitrogen is directly linked to the sulphur of the sulphonamide group of formula (I).

9. A compound according to any of claims 1 to 8, wherein A is T or T-NH- and T is optionally substituted with up to 3 R⁷, independently being F; Cl; CH₃; OCH₃; N(CH₃)₂; T¹; OT¹; or NHT¹.

10. A compound according to claim 9, wherein T¹ is phenyl, which is optionally substituted with up to three halogens independently being F; or Cl.

11. A compound according to any of claims 1 to 10, wherein T² is phenyl, which is unsubstituted or substituted with up to three R¹⁶, which are the same or different.

12. A compound according to claim 11, wherein R¹⁶ independently being F, Cl, COOR²², C(O)NR²²R^{22a}, NR²²R^{22a} or CF₃.

13. A pharmaceutical composition comprising a compound or a pharmaceutically acceptable salt thereof according to any of the claims 1 to 12 together with a pharmaceutically acceptable carrier, optionally in combination with one or more other pharmaceutical compositions.

14. A pharmaceutical composition according to claim 13, comprising one or more additional compounds or pharmaceutically acceptable salts thereof selected from the group consisting of compounds according to any of the claims 1 to 12 and not being the first compound; other 11β-HSD 1 inhibitors; DPP-IV inhibitors; insulin sensitizers selected from the group consisting of PPAR agonists and biguanides; insulin and insulin mimetics; sulfonylureas and other insulin secretagogues; α-glucosidase inhibitors; glucagon receptor antagonists; GLP-1, GLP-1 mimetics, and GLP-1 receptor agonists; GIP, GIP mimetics, and GIP receptor agonists; PACAP, PACAP mimetics, and PACAP receptor 3 agonists; cholesterol lowering agents selected from the group consisting of HMG-CoA reductase inhibitors, sequestrants, nicotinyl alcohol, nicotinic acid or a salt thereof, PPARγ agonists, PPARα/γ dual agonists, inhibitors of cholesterol absorption, acyl CoA: cholesterol acyltransferase inhibitors, and anti-oxidants; PPAR agonists; antiobesity compounds ; ileal bile acid transporter inhibitors; anti-inflammatory agents; protein tyrosine phosphatase-1B (PTP-1B) inhibitors; anti-hypertensive compounds; and compounds for the treatment of cardiovascular diseases, particularly atherosclerosis.

15. A compound or a pharmaceutically acceptable salt thereof according to any of claims 1 to 12 for use as a medicament.

16. Use of a compound or a pharmaceutically acceptable salt thereof according to any of claims 1 to 12 for the manufacture of a medicament for the treatment or prophylaxis of non-insulin dependent diabetes mellitus, hyperglycemia, low glucose tolerance, insulin resistance, β-cell dysfunction, obesity, lipid disorders, dyslipidemia, hyperlipidemia, hypertriglyceridemia, hypercholesterolemia, low HDL levels, high LDL levels, atherosclerosis and its sequelae, vascular restenosis, pancreatitis, abdominal obesity, neurodegenerative disease, retinopathy, nephropathy, neuropathy, Syndrome X, Alzheimer's disease, HAART-associated lipodystrophy or other conditions and disorders where insulin resistance is a component or that may be treated by inhibition of the 11β-HSD1 enzyme.

17. A method for treating, controlling, delaying or preventing in a mammalian patient in need of treatment one or more conditions selected from the group consisting of the onset of non-insulin dependent diabetes mellitus, hyperglycemia, low glucose tolerance, insulin resistance, β-cell dysfunction, obesity, lipid disorders, dyslipidemia, hyperlipidemia, hypertriglyceridemia, hypercholesterolemia, low HDL levels, high LDL levels, atherosclerosis and its sequelae, vascular restenosis, pancreatitis, abdominal obesity, neurodegenerative disease, retinopathy, nephropathy, neuropathy, Syndrome X, Alzheimer's disease, HAART-associated lipodystrophy and other conditions and disorders where insulin resistance is a component or that may be treated by inhibition of the 11β-HSD 1 enzyme, wherein the method comprises the administration to said patient of a therapeutically effective amount of a compound according to any of the claims 1 to 12 or a pharmaceutically acceptable salt thereof.

18. A process for the preparation of a compound according to any of the claims 1 to 12, comprising the step of
coupling a compound of formula (II) 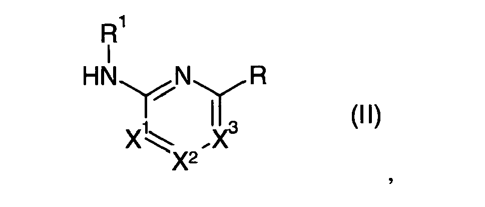 wherein R is T²,
with a compound of the formula A-S(O)₂-Cl, wherein A is T or C₁₋₆ alkyl; or
with a compound of the formula A-H and SO₂Cl₂, wherein A is T-N(R^{1a})-.
